Europäisches Patentamt

**European Patent Office**

Office européen des brevets

(11) Publication number : **0 662 612 A1**

# EUROPEAN PATENT APPLICATION

(21) Application number : **95400021.2**

(22) Date of filing : **05.01.95**

(51) Int. Cl.[6] : **G01N 33/574**

(30) Priority : **05.01.94 IL 10826993**

(43) Date of publication of application :
**12.07.95 Bulletin 95/28**

(84) Designated Contracting States :
**BE DE FR GB IT**

(72) Inventor : **Shalitin, Channa**
**70, Pinsker St.**
**Haifa (IL)**

(74) Representative : **Gillard, Marie-Louise et al**
**Cabinet Beau de Loménie**
**158, rue de l'Université**
**F-75340 Paris Cédex 07 (FR)**

(71) Applicant : **Shalitin, Channa**
**70, Pinsker Street**
**Haifa (IL)**

(71) Applicant : **Technion Research &**
**Development Foundation Ltd.**
**Technion City**
**Haifa 32000 (IL)**

(54) **A method of using novel antibodies for the detection of benign and malignant tumors.**

(57) A novel ELISA type, referred to as Sandwich ELISA comprising polyclonal anti-p21 antibodies generated from a p21 containing cysteine residues is described. The Sandwich ELISA type was used for the detection of anti-non-ras-p21 antibodies with a particular N-terminal aminoacid sequence of two peptides. These antibodies are used at the same time as catcher and detector of 21 KDA protein, the multivalent p21 antigen being bound to the catcher at a pH of 9.6. The ELISA procedure using the Sandwich type was found to be most useful for clinical monitoring of patients with various types of tumors.

EP 0 662 612 A1

The invention relates to a novel type of ELISA, hereafter named Sandwich Elisa and its use for the detection of non-ras-p21 protein in biological fluids of benign and malignant tumor bearing patients.

## BACKGROUND OF THE INVENTION

In our previous European Patent Application N° 92116788.8 (published under N° 0 535 663) there were described purified and isolated anti-non-ras antibodies which bind a non-ras p21 protein having a molecular weight of 21 KDa. These antibodies were found to be able to detect malignant diseases by their reaction with biological fluids of patients with tumors. The method used is based on a modified ELISA procedure, which enables a clinical monitoring of patients with malignancies lacking established tumor markers, such as renal cell carcinoma, seminomatus germ cell tumors and bladder carcinoma. In this manner, it is possible to determine whether a residual cancer remained after a certain treatment.

Although the method is quite simple and sometimes gave reliable results, there are many cases where the direct ELISA might be inhibited by serum interference factors.

Furthermore, up to now there are no established tumor markers which are capable to measure response to therapy in malignant lymphomas.

It is an object of the present invention to provide a novel type of ELISA, hereafter referred to Sandwich ELISA. It is another object of the present invention to provide a method which enables to assess the level of p21 in sera with malignant diseases, before and after therapy using the Sandwich ELISA type test with substantially no false-positive results.

## BRIEF DESCRIPTION OF THE INVENTION

The invention relates to a novel type of Sandwich ELISA, comprising polyclonal anti-p21 antibodies generated from a 21 kDa protein containing cysteine residues which forms homomultimers or heteromultimers with other cysteine containing polypeptides via disulfide bonds, wherein the said polyclonal antibodies serve at the same time as catcher and detector of the 21 kDa protein, the multivalent p21 antigen being bound to the catcher at a pH of 9.6. The novel type of Sandwich ELISA was found to be useful as a method of assessing an accurate level of p21 in sera of patients with malignant diseases before and after therapy, being correlated with the clinical course. It was found that the Sandwich ELISA test according to the present invention, does give a more reliable result in respect to patients with active disease and healthy controls.

In this manner, the anti-p21 Sandwich ELISA test is most valuable in monitoring a treatment and in detection of recurrences in patients with malignant diseases. Using the Sandwich ELISA method it was possible to correlate serum p21 levels with disease activity in 72 % of the patients with malignant lymphomas.

## DESCRIPTION OF THE FIGURES

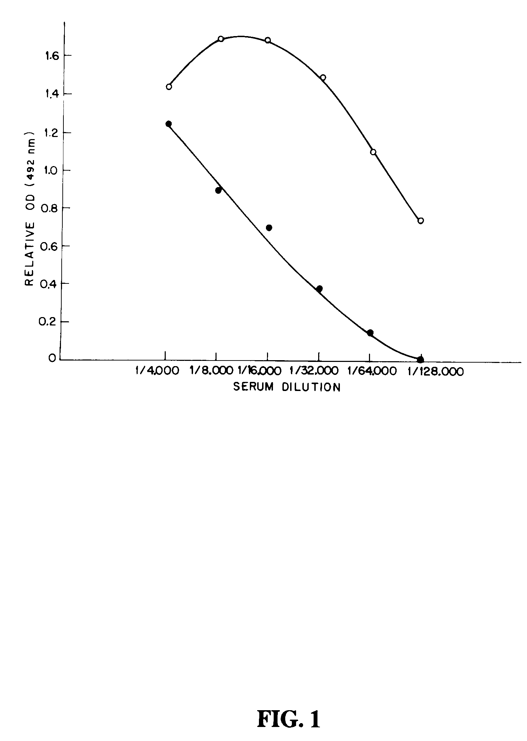

FIGURE 1 : presents the serum p21 titration curve assayed by direct ELISA (o), or by Sandwich ELISA (•). The data were obtained as follows : aliquots of serum from a lymphoma patient were diluted in a coating buffer at pH of 9.6 and p21 levels assayed by direct ELISA (at pH of 7.4, as previously described) and by the Sandwich ELISA (at pH 9.6) as in the present invention. The unexpected result caused by the change of pH may be explained by the dissociation of the S-S bridges of the antigen at pH 9.6.

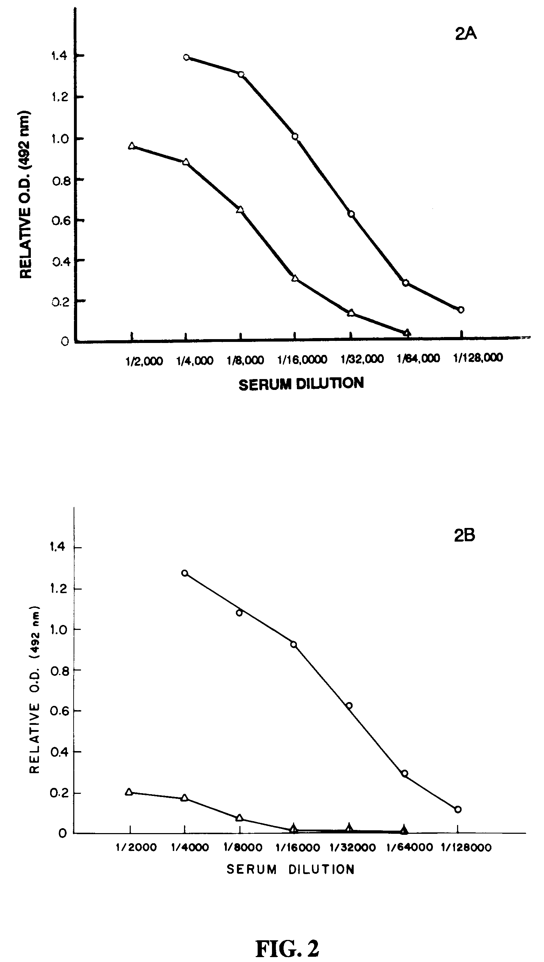

FIGURE 2 : illustrates in a clear manner, the influence of the pH of 7.4 (2A) as used in the standard ELISA and pH of 9.6 (2B) as used in the present invention on the antigen binding in the Sandwich ELISA, wherein :
o = serum of lymphoma patient, and
$\Delta$ = serum of healthy control.

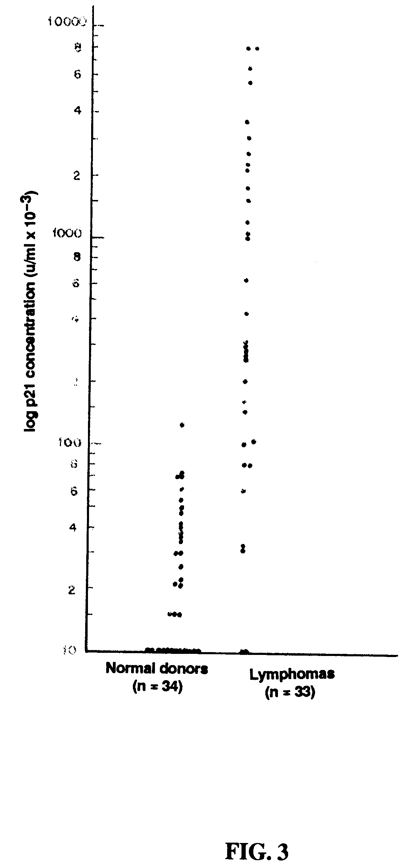

FIGURE 3 : the assay of p21 serum levels in sera of normal patients, as compared with pre-treatment lymphoma patients. Sera from 34 healthy donors were compared with sera of 33 lymphoma patients with active disease. The p21 levels were assayed by Sandwich ELISA. The data presented are mean of two determinations.

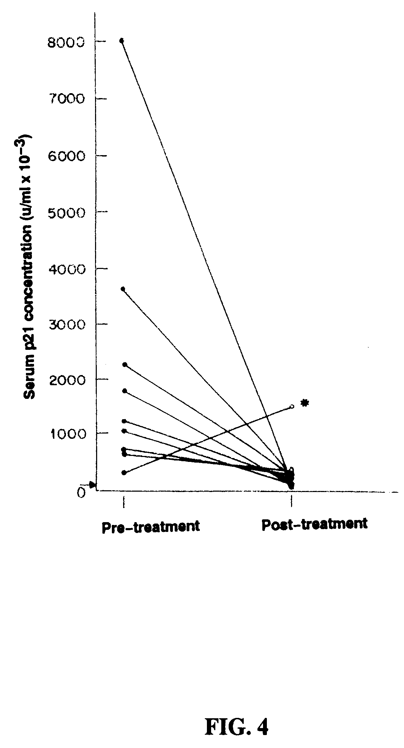

FIGURE 4 : the effect of therapy on the level of p21 in sera of 9 lymphoma patients with increased pre-treatment p21 levels. The p21 levels were assayed by Sandwich ELISA before (•) and after (o) the initiation of chemotherapy + radiotherapy (3 to 14 months). The arrow indicates the upper level of the normal range and *, patients suffering from progressing disease. The rest of the patients achieved complete remission.

## DETAILED DESCRIPTION OF THE INVENTION

The method for the detection of benign and malignant tumors, using the purified and isolated anti-non-ras-p21 antibodies was carried out using the Sandwich ELISA. As known, the Sandwich ELISA, also known as an immunoenzymometric assay, is applicable to bivalent or polyvalent antigens. In this assay, the solid-phase immobilized antibody is incubated with the standard or test antigen. According to the present invention, the Sandwich ELISA uses polyclonal anti-p21 antibodies as catcher antibodies as well as detector antibodies.

The p21 contains cysteine residues which form homomultimers or heteromultimers with other cysteine containing polypeptides via disulfide bonds, since p21 was released from complexes of high molecular weight only by reducing agents.

Thus, according to one of its feature, the present invention relates to a sandwich ELISA-type assay for the determination of p21 antigens in a sample wherein polyclonal anti-p21 antibodies are used both to capture and to detect the said p21 antigens, the p21 antigens being bound to the capture antibodies at a pH of 9.6.

Generally, the polyclonal anti-p21 antibodies used as detecting antibodies are labeled with an enzyme label.

Preferably, the enzyme label is peroxidase. In this case, the activity of the enzyme is developed by hydrogen peroxide and a conventional chromogen, for example o-phenylenediamine.

The p21 antigens are generally specific of tumoral cells, for example those of renal cell carcinoma, bladder carcinoma, prostate carcinoma, testicular germ-cell tumors, ovary carcinoma, malignant lymphomas, gastrointestinal lymphomas or liver tumors.

According to another of its feature, the present invention relates to a kit for use in the above-described assay, said kit essentially comprising (i) a solid support to which are bound polyclonal anti-p21 antibodies, (ii) a solution of polyclonal anti-p21 antibody labeled with an enzyme label and (iii) a reagent for diluting the sample to be assayed at a pH of 9.6. This kit may further comprises one or more solutions providing reagents for coupling the detecting antibody to the enzyme and/or developing the activity of the enzyme.

As can be noticed from Figure 1, there is a significant difference in the linearity at serum dilutions.

According to the invention, the multivalent p21 antigen is bound to the catcher antibodies at a pH of 9.6 (•) and not as usually done in PBS with the normal ELISA at a pH of 7.4 (o). Whereas in case of Sandwich ELISA, this linearity exists for a serum dilution in the range of between 1/4,000 to 1/128,000, the direct ELISA shows a linearity only in the range of 1/16,000 to 1/128,000.

As a consequence, the use of Sandwich ELISA will give more reliable results.

In Figure 3, it is shown that serum p21 levels as determined by Sandwich ELISA in 34 healthy controls, were $27.3 \pm 28.7 \times 10^{-3}$ u/ml (mean $\pm$ SD). These values were considerably lower than those determined by the direct ELISA ($200 \pm 125 \times 10^{-3}$ u/ml) (not shown).

In the following Table 1, are presented the characteristics of 33 lymphoma patients.

## TABLE 1

Sex :    Male    21

Female    12

Age :    Median    50

Range    20-87

Histology :

| Hodgkin's disease | non-Hodgkin's disease |
|---|---|
| Nodular sclerosis : 7 | Low grade : 7 |
| Mixed cellularity : 2 | Intermediate and high grade : 14 |
| Unknown : 2 | Unknown : 1 |

| Stage       | Systemic Symptoms |    |
|-------------|-------------------|----|
| IA : 6      | A                 | 29 |
| IIA : 11    | B                 | 4  |
| IIB : 1     |                   |    |
| IIIA : 3    |                   |    |
| IIIB : 2    |                   |    |
| IVA : 7     |                   |    |
| IVB : 1     |                   |    |
| Relapse : 2 |                   |    |

The levels of serum p21 as determined by the Sandwich ELISA in the 33 patients, as in the above Table 1, were $1,566 \pm 2,298 \times 10^{-3}$ u/ml (mean $\pm$ SD) [in the range O-8,000 x $10^{-3}$ u/ml]. Obviously, 24 from the 33 patients, i.e. about 72 %, exhibited a significant increase -more than 2SD above the normal mean- in pre-treatment serum levels of p21. The difference between healthy controls and cancer patients is 3 to 80-fold elevated p21 serum levels in lymphoma patients. By contrast, this difference is 3 to 5-fold with the normal ELISA (pH 7.4). Among the malignant diseases which can be assessed using the Sandwich ELISA, according to the present invention the following may be mentioned renal cell carcinoma, bladder carcinoma, prostate carcinoma, testicular germ-cell tumors, ovary carcinoma, malignant lymphomas and gastrointestinal and liver tumors.

In Figure 4, there are presented the results on the level of p21 in sera of 9 patients with malignant lymphomas, assessed before and after therapy and correlated with the clinical course. Among the representative lymphoma patients tested with higher than normal pre-treatment levels of serum p21, the p21 levels decreased to normal in 8 patients who achieved a complete remission after 3 to 14 months following chemotherapy and radiotherapy. However, an increased level of p21 was detected in 1 untreated patient at relapse after 10 months. The p21 serum level remained low or undetectable after a successful therapy in 4 patients who had normal or undetectable pretreatment serum p21 levels.

The invention will be hereinafter illustrated by the following Example presented for a better understanding of the invention, without implying any limitation, being understood that a person skilled in the art, after reading the present specification will be in a position to replace one or more of the reagents, without changing the scope of the invention as covered by the appended claims.

**EXAMPLE** :

**Serum samples** : serum samples were collected between June 1991 and October 1993 at the Department of Oncology, Rambam Medical Center, Haifa. Fifty six samples were taken from 33 patients with primary Hodgkin's lymphoma (22 pts) at presentation and 3-24 months after the initiation of chemotherapy and radiotherapy. The mean ages were $48.6 \pm 19.1$ years (range 20-87 years). Also, 34 normal donors, mean ages $40.6 \pm 14.0$ years (range 18-84 years) were analysed. At the time that the samples were obtained 31 newly diagnosed patients were not yet treated, and 2 relapsed patients had been off therapy for at least 3 months.

The stages according to the Ann Arbor Classification (Carbone PP et al. Cancer Research 31:1860 (1971)) were IA, IB, IIA, IIB, IIIA, IIIB, IVA and IVB. Staging was based on findings on physical examination, laboratory routine tests, imaging procedures (computed tomography scans of chest and abdomen, and gallium scans), and bone marrow biopsy. Patients were treated with chemotherapy + radiotherapy. Complete remission was defined as the disappearance of all clinical evidence of active tumor. Disease progression was considered as an increase of 25 % or more in size of pre-existing lesions or appearance of new lesions.

Sera were stored at -20°C until used. Just before the assay, sera were heated 30 minutes at 56°C to inactivate complement and a serum interference factor.

**Anti-p21 polyclonal antibodies** : anti-p21 polyclonal antibodies were produced by immunization of rabbits using p21 protein isolated by SDS-polyacrylamide gel electrophoresis under reducing conditions.

Specifically, rabbits were immunized using 10 µg portions of p21 peptide in PBS emulsified in Freund's complete adjuvant and injected intradermally into rabbits at multiple sites. The rabbits were given four booster injections with 10 µg peptide in FCA at 4-5 weeks intervals, and were bled two weeks later (Shalitin et al., Int. J. Oncol., 1, 107-112, 1992).

**Sandwich ELISA method** : Polyvinyl microtiter plates (Costar) were coated with 200 µl/well anti-p21 antiserum as catcher antibodies (at a 1000-fold dilution in PBS pH 7.4 containing 2 % wt/vol, nonfat dry milk (Cadbury's Marvel)) at 4°C overnight. Plates were washed three times with TBST (200 μl/well, 50mM Tris-HCl pH 8.0 and 150mM NaCl containing 0.05 % Tween® 20) and the excess detergent was removed by further washing the plates with TBS (200 μl/well, 50 mM Tris-HCl pH 8.0, 150 mM NaCl) three additional times.

The plates were found to be stable for 4 weeks at room temperature or for one week at 37°C.

Normal and cancer patients' sera were serially diluted (1/2000-1/16000) with carbonate buffer (25mM $Na_2CO_3$, 55mM $NaHCO_3$, pH 9.6 containing 1mM PMSF) and incubated at 37°C for 60 minutes or overnight at 4°C. Plates were washed twice with phosphate buffered saline (pH 7.4).

Plates were coated for 90 minutes at room temperature with PBS containing 2 % (wt/vol) nonfat dry milk. Antigen capture plates were incubated 60 minutes at 37°C with 100 μl/well of anti-p21 PAb at 1/1000 dilution in PBS containing 2 % (wt/vol) nonfat dry milk. Then the plates were washed three times with TBST (200 μl/well). Antigen-antibody complexes were detected by incubation with biotinylated goat anti-rabbit IgG (from Sigma) (1/1000 in TBST) for 30 minutes at room temperature. The plates were washed three times with TBST. Finally, Extra Avidin Peroxidase (from Sigma) 1/500 in TBST was added for 30 minutes at room temperature and the plates were washed three times with TBST. The solid phase bound enzyme was detected using a chromogenic substrate (100 μl/well) containing 0.05 % o-phenylenediamine and 0.05 % hydrogen peroxide in 0.1M phosphate citrate buffer pH 5.0. After 10-30 minutes, the reaction was stopped by the addition of 50 μl of 2N $H_2SO_4$ and color yield was assessed at 492nm using Anthos Labtech 2001 reader. Negative controls included buffer instead of sera to measure non specific binding of avidin-biotinylated peroxidase complex which yielded a reading of 0.1-0.2 A492. Serum p21 values were calculated from a linear calibration curve obtained from a positive serum sample yielding a reading of 0.8-0.9 A492 for 100 arbitrary units of p21 per ml at dilution of 1/10,000.

## Claims

1. A sandwich ELISA-type assay for the determination of p21 antigens in a sample wherein polyclonal anti-p21 antibodies are used both to capture and to detect the said p21 antigens, the p21 antigens being bound to the capture antibodies at a pH of 9.6.

2. An assay according to claim 1, wherein the detecting antibodies carry an enzyme label.

3. An assay according to claim 2, wherein the enzyme label is peroxidase and wherein the activity of the enzyme is developed by hydrogen peroxide and a chromogen such as orthophenylenediamine.

4. An assay according to any one of claims 1-3, wherein the p21 antigens are characteristic of tumoral cells.

5. An assay according to claim 5, in which the tumoral cells are those of renal cell carcinoma, bladder carcinoma, prostate carcinoma, testicular germ-cell tumors, ovary carcinoma, malignant lymphomas, gastrointestinal lymphomas or liver tumors.

6. A kit for use in the assay of any one of claims 1-5, which essentially comprises :
   - a solid support to which are bound polyclonal anti-p21 antibodies ;
   - a solution of polyclonal anti-p21 antibody labeled with an enzyme label ; and
   - a reagent for diluting the sample to be assayed at a pH of 9.6.

**FIG. 1**

FIG. 2

**FIG. 3**

**FIG. 4**

**European Patent Office**

## EUROPEAN SEARCH REPORT

Application Number

| | DOCUMENTS CONSIDERED TO BE RELEVANT | | EP 95400021.2 |
|---|---|---|---|
| Category | Citation of document with indication, where appropriate, of relevant passages | Relevant to claim | CLASSIFICATION OF THE APPLICATION (Int. Cl. 6) |
| D,Y | EP - A - 0 535 663 (TECHNION RESEARCH & DEVELOPMENT FOUNDATION LTD.) * Abstract; claims 1,3,4; page 4, lines 28-56; page 5; page 8, line 53 - page 9, line 18 * | 1-6 | G 01 N 33/574 |
| Y | US - A - 4 737 453 (F.J. PRIMUS) * Claims 1-5,9,11,14,15 * | 1-6 | |
| A | EP - A - 0 214 520 (ONCOGENE SCIENCE, INC.) * Claims 1,4-6,25-27,38,40, 41,46,52,65,67,69,72,75,81, 82,84,86,87 * | 1-6 | |
| | | | TECHNICAL FIELDS SEARCHED (Int. Cl.6) |
| | | | G 01 N 33/00 |

The present search report has been drawn up for all claims

| Place of search | Date of completion of the search | Examiner |
|---|---|---|
| VIENNA | 24-02-1995 | MAZZUCCO |